# EUROPEAN PATENT APPLICATION

(11) **EP 2 839 840 A1**
(43) Date of publication of application: **25.02.2015**
(21) Application number: 12870062.2
(22) Date of filing: 11.05.2012
(51) Int. Cl.: A61K 39/145, A61P 31/16, C12N 15/44, C07K 16/10, C07K 14/11

(54) **RECOMBINANT TRIVALENT VACCINE AGAINST HUMAN INFLUENZA**

(30) Priority: 28.02.2012 RU 2012107028
(71) Applicant: Obschestvo s Ogranichennoi Otvetstvennostju "NTpharma", Moscow 115114 (RU); Ataullakhanov, Rustam Ravshanovich, Moskva 119019 (RU)
(72) Inventor: ATAULLAKHANOV, Rustam Ravshanovich, Moskva 119019 (RU); SHMAROV, Maksim Mikhailovich, Moscow 127427 (RU); SEDOVA, Elena Sergeevna, Shikhany 412950 (RU); LOGUNOV, Denis Jurievich, Moscow 123098 (RU); PICHUGIN, Aleksey Vasilievich, Moscow 119607 (RU); ATAULLAKHANOV, Ravshan Inoyatovich, Moscow 123104 (RU); KHAITOV, Rakhim Musaevich, Moscow 123056 (RU)
(74) Representative: Sterlina, Tatjana
(86) International application number: PCT/RU2012/000366
(87) International publication number: WO 2013/129961

(57) **Abstract**

The invention relates to medicine, in particular to prevention of infectious diseases of the respiratory tract in humans, including influenza virus by vaccines. Recombinant trivalent vaccine against human influenza contains recombinant adenoviral vector with a nucleotide sequence which encodes at least one antigen of influenza virus strain, while non-replicating nanoparticles are used as adenoviral vectors based on genome of human adenovirus of serotype 5, capable of expressing the haemagglutinin of influenza virus which induce human immune response to influenza virus, wherein the vaccine contains composition of three kinds of non-replicating nanoparticles, each of which carries different influenza hemagglutinin genes, further comprises immunostimulant and formulating buffer. In this solution the influenza virus hemagglutinin genes are: H1, H3, HB and any other recommended by the World Health Organization at the time of production. Formulating buffer is added to the composition to achieve a total volume of 0.5 ml and a vaccine dose of 0.5 ml. Recombinant trivalent vaccine contains:
- Non-replicating nanoparticles with inset of hemagglutinin H1 not more than 10⁷ PFU;
- Non-replicating nanoparticles with inset of hemagglutinin H3 not more than 10⁷ PFU;
- Non-replicating nanoparticles with inset of hemagglutinin HB not more than 10⁷ PFU;
- Immunostimulant 1 - 20 µg

Peptidoglycans are used as immunostimulants, and acidic peptidoglycan having a molecular weight of from 1,200 to 40,000 kDa is used as peptidoglycan. In another embodiment, peptidoglycan of bacteria cell wall is used as a peptidoglycan. Recombinant trivalent vaccine is administered intranasally.

## Description

### FIELD OF THE INVENTION

Innovation refers to healthcare, in particular to a vaccination-based prevention of human respiratory tract infectious diseases including influenza.

### BACKGROUND OF THE INVENTION

Influenza is a highly contagious viral disease of humans, birds and animals. Seasonal influenza (epidemics) of virus type A or B, leading to increased mortality and significant economic damage are recorded each year in the world. Usually, the most serious diseases are caused by type A influenza viruses, of which the subtypes A(H1N1) and A(H3N2) are currently circulating among humans. These viruses are widespread in nature and can infect humans, many animal species (horses, pigs, seals, etc.) and birds; they can constitute pandemic threat with many human losses (Gorman O.T. et al. Evolutionary processes in influenza viruses: divergence, rapid evolution, and stasis.// Curr. Top. Microbiol. Immunol., 1992, Nº 176, c. 75-97; and "Influenza. The information bulletin (in Russian)" at http://www.who.int/mediacentre/factsheets/fs211/ru/index.html).

Protection against the constant attacks of influenza viruses is complicated by the difficulty of predicting the emergence of new pandemic and epidemic strains, and low cross-reactivity of vaccines, most of which only provide good protection from influenza virus strain out of which they were derived.

WHO proposes to conduct works in three areas to solve the problem of protection of maximum number of people against avian influenza in the event of a pandemic caused by this virus: increasing the use of vaccines against seasonal influenza, increasing production capacity to manufacture large amounts of vaccine in the event of a pandemic, and holding research on the production of new efficient vaccines, including obtaining new types of vaccines, such as recombinant subunit vaccines, DNA vaccines and viral vaccines based on the viral vectors and virus-like particles (Abdulhaqq S.A.,Weiner D.B. DNA Vaccines: developing new strategies to enhance immune responses. Immunol. Res., 2008, 42, 219-232).

Up to date one of the most effective approach for solving the abovementioned problems is the use of genetic vaccines based on adenovirus vectors (Zaia JA. The status of gene vectors for the treatment of diabetes.// Cell Biochem Biophys. 2007; 48(2-3):183-90). The penetration of genetic material into cells and target protein pathogen gene expression occurs in them in case of introducing of such vaccines into the body. As a result, the corresponding antigens of pathogens are recognized by the immune system that results in the induction of both humoral and cellular immune response (A. Karpov et al., Construction of recombinant avian adenoviruses CELO which express genes of gB, gE, gl glycoproteins of Marek's disease viruses (in Russian), Biotechnology, 2007, Nº 5, p. 38-44) (Knoblich H.V. et al., Antibody titers to infectious bursal disease virus in broiler chicks after vaccination at one day of age with infectious bursal disease virus and Marek's disease virus, Avian Dis., 2000, Nº 44 (4), p. 874-84).

To date, a recombinant human adenovirus type 5 is the most studied and frequently used for genetic immunization. Vaccines based on recombinant human adenovirus type 5 have several advantages against other genetic vaccines. Firstly, the recombinant adenoviruses are replication-defective and cannot cause diseases. The safety of human adenovirus type 5 with E1/E3 - deleted genome regions is confirmed by a number of various conducted clinical trials of vaccines and therapeutic drugs based on them (Hoelscher M.A. et al., Development of adenoviral-vector-based pandemic influenza vaccine against antigenically distinct human H5N1 strains in mice, Lancet, 2006, Nº 367 (9509), c. 475-81) (Van Kampen K.R. et al., Safety and immunogenicity of adenovirus-vectored nasal and epicutaneous influenza vaccines in humans, Vaccine, 2005, Nº 23(8), p. 1029-36). Secondly, recombinant adenoviruses can be administered intranasally, and as a result, induce mucosal immune response. Thirdly, at the moment a rapid and flexible technology for producing recombinant adenoviruses are developed allowing large scale production of various candidate vaccines based on adenovirus vectors on one production line without its re-equipment and manufacturing instructions change. The abovementioned features make recombinant adenoviruses a good technology platform for creating a wide range of vaccines against different subtypes of influenza virus.

Recombinant adenoviruses have the following characteristics (Harrop R. et al., Recombinant viral vectors: cancer vaccines, Adv. Drug Deliv. Rev., 2006, Nº 58(8), p. 931 - 47).
- able to transduce both dividing and post-mitotic cells (Tang D.C. et al., Overexpression of adenovirus-encoded transgenes from the cytomegalovirus immediate early promoter in irradiated tumor cells, Hum. Gene Ther., 1997, Nº 8(17), p. 2117-24) (Tang D. C. Et al., Butyrate - inducible and tumor - restricted gene expression by adenovirus vectors, Cancer Gene Ther., 1994, Nº 1 (1), pp.15-20.);
- DNA adenovirus remains in the extrachromosomal form;
- can be obtained at a titer higher than 10¹⁰ PFU/ml, allowing to use them as live recombinant vaccines for veterinary and human medicine;
- able to replicate only *in vitro* in specific cell lines;
- provide a high level of target gene expression in the target cell;
- excreted for 4-5 weeks;
- Immunization with recombinant adenoviruses provides induction of both cellular and humoral immune response (Schagena F.H.E. et al., Immune responses against adenoviral vectors and their transgene products: a review of strategies for evasion, Critical Reviews in Oncology/Hematology, 2004, Nº 50(1), p. 51-70);
- the process of obtaining a new recombinant adenovirus takes several weeks, allowing to quickly react to the changing epidemiological situation in the shortest amount of time possible.

Intranasal immunization with human recombinant adenovirus type 5 does not cause any adverse effects (Van Kampen K.R. et al., Safety and immunogenicity of adenovirus-vectored nasal and epicutaneous influenza vaccines in humans, Vaccine, 2005, Nº 23(8), p. 1029-36).

Application of genetic vaccines based on human adenovirus type 5 may be limited by the presence of preexisting immunity in people already exposed to this virus (Bangari D.S. et al., Comparative transduction efficiencies of human and nonhuman adenoviral vectors in human, murine, bovine, and porcine cells in culture, Biochem. Biophys. Res. Commun., 2005, Nº 327, p. 960-966).

However, unlike parenteral administration, the intranasal immunization with a vaccine based on human adenovirus type 5 one can avoid exposure to preexisting immunity (Van Kampen K.R. et al., Safety and immunogenicity of adenovirus-vectored nasal and epicutaneous influenza vaccines in humans, Vaccine, 2005, Nº 23(8), p. 1029-36). High immunogenicity of recombinant human adenovirus type 5 in case of intranasal administration leads to efficient delivery of the transgene through the mucosal barrier. It was shown that even in the single administration of vaccines based on human adenovirus type 5 leads to prolonged ex vivo expression of the transgene, despite preexisting immunity as in laboratory animals (Wadsworth S.C. et al., Adenovirus vector-infected cells can escape adenovirus antigen-specific cytotoxic T-lymphocyte killing in vivo, J. Virol., 1997, Nº 71(7), p. 5189 - 51 96). Similarly in primates (Zabner J. et al. Safety and efficacy of repetitive adenovirus-mediated transfer of CFTR cDNA to airway epithelia of primates and cotton rats, Nat. Genet, 1994, Nº 6(1), p. 75-83).

Modern level of vaccine formulations involves not only the induction of protective specific immunity, but also induction of innate immunity to a wider range of anti-infectious protection. Ones of the key receptors of the animal innate immune system are Toll-like receptors (TLR). Receptors of this family are able to recognize specific highly conserved molecular regions (patterns) in the pathogen structure initiating the reactions development of both innate and adaptive immunity resulting in pathogen elimination from the body. In addition, each separate type of TLR is able to bind a wide range of molecules of microbial origin with different chemical properties and structure. Thus, the data obtained today allow to increase the immunogenicity of the vaccine antigen when combined with substances capable to interact with pattern-recognizing receptors, activation of which leads to additional stimulation of immune responses. Prospect of this approach is supported by numerous clinical trials of various ligands of pattern-recognizing receptors as molecular adjuvants for vaccines (O'Hagan D.T., MF59 is a safe and potent vaccine adjuvant that enhances protection against influenza virus infection MF59, Expert Rev. Vaccines, 2007, Nº 6(5), 699), (Di Paolo D. et al., One-year vaccination against hepatitis B virus with a MPL-vaccine in liver transplant patients for HBV-related cirrhosis, Transplint., 2010, Nº 23(11), p. 1105).

WHO recommendations which describe the advantages of using seasonal trivalent vaccines, including H1N1, should be taken into account in development of vaccines (Pandemic influenza H1N1, 2009, Short Message No 23, August 10, 2010, Geneva, available: http://www.who.int/csr/disease/swineflu/ notes/briefing 20100810/ru/index.html).

Thus, it is necessary to create highly immunogenic intranasal vaccines of single administration, which are able to simultaneously protect against different strains of influenza virus and other infectious agents of respiratory diseases in humans, for effective protection against respiratory infections.

Vaccine against pandemic strains of influenza viruses is known based on recombinant adenoviral vector comprising a nucleotide sequence, which encodes at least one antigen of the avian influenza virus strain (US Patent Application No 2008/0187557).

Significant deficiency of this vaccine is the presence of avian influenza antigens only (H5N1 strain, H7N7 strain or H9N2 strain), what significantly narrows the range of its specificity and does not allow its use for the prevention of seasonal human influenza caused by different subtypes of H₁ as well as other respiratory infectious diseases in humans. This vaccine is chosen by the authors as an alternative.

Also a pharmaceutical composition is known containing at least 4 vectors, each vector being capable of expressing the haemagglutinin or immunogenic peptides of different influenza virus strains. This vaccine is able to induce immune response in humans to target influenza virus strains (US Patent Application No 2010/00987221).

Plasmids and various viral vectors may be the vector in this technical solution, while the composition may contain adjuvants. This solution was taken by the authors as a prototype.

The deficiency of this composition is the need of booster (repeated) vaccination to obtain a protective immunity to strains of influenza virus due to the influence of antigenic competition of a large number of antigens in vaccine composition on immunogenicity of each of them. Furthermore, the vaccine is not capable of activating the innate immunity and therefore does not induce broader immune protection against other species of microorganisms.

### SUMMARY OF THE INVENTION

The object of the present invention is to provide a 3-valent vaccine, which in a single intranasal administration will provide a protective immunity in humans within 6 months or more to all the influenza virus strains in vaccine composition; reduced cost of production and the cost of drugs, medical instruments and working time of medical staff while providing the opportunity for rapid creation of seasonal epidemiologically significant vaccine strains, reduction of vaccine reactogenicity; providing a wide spectrum of antimicrobial prophylactic vaccine activity against infectious diseases of the respiratory tract.

The problem is solved by the fact that recombinant trivalent human influenza vaccine against contains recombinant adenoviral vector with a nucleotide sequence that encodes at least one strain of influenza virus strain antigen, while in this case as adenoviral vector are used non-replicating nanoparticles based on the genome of human adenovirus type 5 able to express haemagglutinin of influenza virus which induces an immune response in human to influenza virus; additionally the vaccine contains composition of three types of non-replicating nanoparticles, each of which carries different hemagglutinin genes of influenza virus, as well as the immunostimulant and formulating buffer. In this solution the hemagglutinin genes of influenza virus are: H1, H3, HB, and any recommended at the time of vaccine production by the World Health Organization. Formulating buffer is added to the composition to achieve a total volume of 0,5 ml, and the vaccine dose is 0.5 ml. Recombinant trivalent vaccine contains:
- Non-replicating nanoparticles with insert of hemagglutinin H1 not more than 10⁷ PFU;
- Non-replicating nanoparticles with insert of hemagglutinin H3 not more than 10⁷ PFU;
- Non-replicating nanoparticles with insert of hemagglutinin HB not more than 10⁷ PFU;
- Immunostimulant 1-20 µg

Peptidoglycans are used as immunostimulant, and acidic peptidoglycan having a molecular weight from 1,200 to 40,000 kDa is used as the peptidoglycan. In another embodiments, peptidoglycan of bacteria cell wall is used as a peptidoglycan. Administration of recombinant trivalent vaccine is made intranasally.

### BRIEF DESCRIPTION OF THE DRAWINGS

Figure 1 shows the results of HAI (Hemagglutination inhibition).
Figure 2 presents survival curves for mice (A) and the change in their weight (B) after infection with a lethal dose of H1N1 influenza virus.
Figure 3 shows the levels of specific antibodies to hemagglutinin of influenza virus H1 N1 defined in HAI assay in sera of mice immunized intranasally with non-replicating nanoparticles in a dose of 10⁷ PFU per animal in conjunction with an immunostimulant in the following doses:
Figure 4 shows the level of specific antibodies to the hemagglutinin of influenza virus H1N1 defined in virus neutralisation test (VNT) in sera of mice immunized intranasally with non-replicating nanoparticles in a dose of 10⁷ PFU per animal in conjunction with an immunostimulant in the following doses.
Figure 5 shows the levels of specific antibodies to hemagglutinin of influenza virus H1N1 defined in HAI analysis in sera of mice immunized intranasally with nonreplicating nanoparticles in a dose of 10⁷ PFU per animal in conjunction with an immunostimulant in a dose of 20 µg per animal.
Figure 6 shows the levels of specific antibodies to hemagglutinin of influenza virus H1N1 defined in VNT in sera of mice immunized intranasally with nonreplicating nanoparticles in a dose of 10⁷ PFU per animal in conjunction with an immunostimulant in a dose of 20 µg per animal.
Figure 7 shows the survival curves of mice immunized with trivalent vaccine after exposure with lethal doses of influenza A viruses - H1N1, H3N2, and influenza B.
Figure 8 shows curves of weight decrease of mice immunized with a trivalent vaccine after exposure with lethal doses of influenza A viruses - H 1 N 1, H3N2, and influenza B.
Figure 9 shows the level curve of specific antibodies to hemagglutinin of influenza virus H1.

### INVENTION IMPLEMENTATION

WHO recommendations were used to solve the tasks, which proposed to conduct works in three areas: increasing the use of vaccines against seasonal influenza, providing opportunities to increase production capacity to manufacture large amounts of vaccine in the event of a pandemic, and holding research on the production of new efficient vaccines, including obtaining new types of vaccines, such as recombinant subunit vaccines based on the viral vectors and virus-like particles. (Abdulhaqq S. A., DNA Vaccines: developing new strategies to enhance immune responses, 2008, Nº 42, p. 219-232).

Protection against the constant attacks of influenza viruses is complicated by the difficulty of predicting the emergence of new pandemic and epidemic strains, and low cross-reactivity of vaccines, most of which only provide good protection from influenza virus strain out of which they were derived, therefore the next task in the development of the vaccine was the task of creating a 3-valent vaccine that provides protective immune response in humans to all influenza virus strains contained in the vaccine, while providing reduced cost of production and the cost of drugs, medical instruments and working time of medical staff.

While providing the opportunity for rapid creation of seasonal epidemiologically significant vaccine strains, reduction of vaccine reactogenicity; providing a wide spectrum of antimicrobial prophylactic vaccine activity against infectious diseases of the respiratory tract.

But the main objective was to enable rapid creation of seasonal epidemiologically significant vaccine strains with reduced vaccine reactogenicity providing a wide spectrum of antimicrobial prophylactic vaccine activity against infectious diseases of the respiratory tract. Besides, such created vaccine should be dosed once and administered intranasally.

Taking into consideration the abovementioned tasks, a safe and economically advantageous highly immunogenic intranasal trivalent recombinant vaccine has been created containing non-replicating nanoparticles based on the adenovirus type 5 genome producing 3 types of epidemiologically significant hemagglutinin of influenza virus directly on nasal mucosa, and possessing the ability to induce the formation of protective immunity in humans after a single dose to all included influenza virus strains, as well as ensuring stimulation of congenital immunity to many microorganisms, besides the vaccine is suitable for the prevention of infectious diseases of the human respiratory tract, particularly influenza.

Hemagglutinin gene H1 can be any hemagglutinin of H1 subtype particularly currently recommended by WHO for vaccine production depending on the epidemiological situation in the world (http:/www.who.int/ru/), e.g., N1 California.

Hemagglutinin gene H3 may be any hemagglutinin of H3 subtype currently recommended by WHO for vaccine production depending on the epidemiological situation in the world (http://www.who.int/ru/), e.g., H1 Perth.

Hemagglutinin gene HB may be any hemagglutinin of HB subtype currently recommended by WHO for vaccine production depending on the epidemiological situation in the world (http://www.who.int/ru/), e.g., HB Brisbane.

The use of the claimed vaccine in the pharmaceutical and clinical practice allows to achieve several technical, medical and economic outcomes:
- the stated vaccine is safe for humans and causes protective immunity to specific influenza virus strains;
- the vaccine is suitable for use as it is administered once intranasally to induce strong immune response for 6 months or more;
- vaccine is suitable for the prevention of human influenza and infectious respiratory diseases of various etiologies;
- The vaccine is economically justified, because a single administration provides prevention of infectious respiratory diseases in humans, and also creates protective immunity to specific influenza virus strains throughout pre-epidemic and epidemic periods of disease.

These unified technical, medical and economic results when practicing the invention of "vaccine" object are achieved by the fact that the claimed vaccine is made using non-replicating nanoparticles based on the genome of human adenovirus type 5 including influenza virus hemagglutinin gene. Peculiarity of the claimed vaccine is that the content of non-replicating nanoparticles expressing multiple influenza virus hemagglutinins (10⁷ PFU of each of the 3 types included in the vaccine) allows to cause the formation of specific protective immunity to each of them with a single intranasal administration, and also provides activation of innate immunity to a broad spectrum of microorganisms which cause respiratory tract diseases. Vaccine prevents human infectious respiratory diseases, particularly influenza.

### EXPERIMENTAL

The following examples reveal:
- Construction of non-replicating nanoparticle based on the genome of human adenovirus type 5 with insertion of hemagglutinin gene of human influenza virus.
- Preparation of the vaccine.
- Determination of the optimal dose of non-replicating nanoparticles.
- Determination of the optimal dose of the immunostimulant.
- Determination of the optimal amount of vaccine volume.
- Identification of immunogenic and protective properties of the trivalent vaccine with the immunostimulant.
- Definition of broad antimicrobial properties of immunostimulant included in the vaccine.

Clinical testing of recombinant trivalent vaccine was carried out on 2 groups of volunteers.

Given tables and figures in the specification confirm the performance of the stated task.

### Example 1.

Construction of non-replicating nanoparticle based on the genome of human adenovirus type 5 with insertion of hemagglutinin gene of human influenza virus.

Construction of nanoparticles based on non-replicating genome of human adenovirus type 5 (size 70-80 nm) with a haemagglutinin gene insert (from the list of: H1, H3, HB) of human influenza virus was performed by homologous recombination in cell culture. A certain generally known recombinant plasmid was taken as a basis, e.g. pJM17 (McGrory W.J. et al., A simple technique for the rescue of early regionI mutations into infectious human adenovirus type 5, Virology, V. 163, Nº 2, 1988). Cloning was performed using conventional laboratory techniques (e.g., Sambrook J. et al., Molecular cloning : a laboratory manual, 3rd ed., Russell, 2001, Vol 1, 2, 3.). Artificially synthesized cDNA influenza hemagglutinin gene (from the list of: H1, H3, HB) was ligated into the general known shuttle plasmid, e.g. pACCMV.pLpA (Roth M. G., Methods in cell biology, Nº 43, p. 175), (Go' mez-Foix A. et al., Adenovirus-mediated transfer of the muscle glycogen phosphorylase gene into hepatocytes confers altered regulation of glycogen metabolism, J. Biol. Chem., 1992, Nº 267 (35), 15, p. 25129-25134). Further on, for mutual transformation of the resulting plasmid pACCMV - H and the plasmid pJM17 they were used to transfect 293 cells (e.g., Nº 300192, CLS, Germany) using the standard technique of calcium phosphate precipitation (Graham F.L. A new technique for the assay of infectivity of human adenovirus 5 DNA, Virology, 1973, Nº52 (2), p. 456-67). Researchers received nanoparticles of non-replicating human adenovirus type 5 with deletions in E1 and E3 regions of the adenovirus genome. In the E1 region the non-replicating nanoparticles contained an expression cassette with the CMV-promoter, influenza virus hemagglutinin gene (from a list: H1, H3, HB) and a polyadenylation signal. That means the researchers obtained three types of non-replicating nanoparticles. Type H1 is selected depending on the seasonal epidemiological situation. H1 California was taken as H1 in the construction period. H3 and HB types are also selected depending on the seasonal epidemiology. H3 Perth and HB Brisbane were taken as H3 and HB (accordingly) in the construction period.

Plaques of non-replicating nanoparticles were formed on cell culture a few days after transfection, they were collected with a Pasteur pipette, the resulting material was propagated on cells of line 293 to obtain 10⁸ PFU of activity per ml of suspension.

### Example 2

### Preparation of the vaccine.

Based on the doses recommended in Examples 3, 4, 5 the contents of each type of non-replicating nanoparticles with insert of influenza virus hemagglutinin gene (H1, H3, HB) in the vaccine generated based on the human adenovirus type 5 genome should not exceed 10⁷ PFU, and immunostimulant content should be from 1 to 20 micrograms per 0.5 ml of vaccine. Preparation of the vaccine is performed in several stages.

Cell suspension prepared in previous example containing non-replicating nanoparticles in the titre 10⁸ PFU per ml was used for further expansion of titles of non-replicating nanoparticles and for preparation of the final vaccine with the stated content of non-replicating nanoparticles of each of the 3 types - not more than 10⁷ PFU, and also with the content of immunostimulant from 1 to 20 µg per 0.5 ml of vaccine.

Thus, the wave bioreactor with 4500 ml suspension of a permissive cell culture 293 was inoculated with 500 ml of cell suspension containing non-replicating nanoparticles with titer 10⁸ PFU per ml for the production of necessary titers of non-replicating nanoparticles.

The suspension was cultured for increase of the number of non-replicating nanoparticles inside cells and achieving their activity of 2 × 10⁸ PFU/ml for approximately 48 hours. After achieving the required content, the cell mass was forwarded for cleaning process, which consisted of several phases:
1) The cell mass was precipitated by centrifugation. The suspension for cleaning was not less than 3,3x10¹¹ PFU per 5 liters. Centrifugation was performed at 6000 g regimen for 15 min and the supernatant liquid was decanted and the remaining solid portion containing cells and non-replicating nanoparticles was transferred to further steps of purification.
2) Removing of non-replicating nanoparticles from the cell culture was carried out by a cell destruction through quadruple process of refreezing and thawing. A pH 8.0 buffer solution was prepared using the following: 5mMTrisHCl, 0.075 MNaCl, 1mMMgCl₂, 5% sucrose, 1% polysorbate 80. Resulted pellet in the previous step was resuspended in 70 ml buffer (content ratio x71). Solution volume was 80 ml.
   Freezing was performed for 2 hours in liquid nitrogen, thawed in a water bath (at +37°C), avoiding overheating.
3) The additional processing with nuclease was performed to facilitate the further removal of genomic cellular DNA. For this purpose benzonase was added to achieve the solution concentration of 150 U/ml and then the solution was placed on a soft stirring using a magnetic stirrer for 3 hours at room temperature (21-23°C).
4) Separation of non-replicating nanoparticles from disrupted cells was performed by centrifugation at 9000g for 10 minutes. The supernatant was collected containing non-replicating nanoparticles.
5) Further purification was carried out by ultrafiltration. For this purpose, the resultant supernatant was diluted with buffer (50mM TrisHCl pH 7.5, 1 M NaCl, 2mM MgCl2, 5% sucrose, pH 7,5) to a volume of 200 ml, stirred with a magnetic stirrer. During the filtration the volume of the circulating solution (retentate) was constantly adjusted to the source volume (200 ml).
6) Further purification was carried out by anion exchange chromatography.
   The retentate was applied to a column (AxiChrom 70/300 with the volume of 400 ml) containing anion exchange sorbent Q Sepharose virus licenced. Nonreplicating nanoparticles are adsorbed on the column, while the impurities are adsorbed and washed with buffer A. After removal of impurities the non-replicating nanoparticles were stripped by washing with buffer solution B. The conditions of chromatography were the following: flow rate 193 ml/min, buffer A (40mM TrisHCl, 0,27 M NaCl, 2mM MgCl₂, 5% sucrose, 0.1% Polysorbate 80, pH 7.5), conductivity was about ∼ 28-30 mS/cm; buffer B (40mM TrisHCl, 0.5M NaCl, 2mM MgCl₂, 5% sucrose, 0.1% polysorbate 80, pH 7.5) conductivity was about ∼ 50 mS/cm. The eluate in the volume of 200 ml was transferred to the next stage.
7) Exclusion chromatography
   Eluate obtained in the previous step was loaded onto the column (AxiChrom 100/300 800 ml) containing sorbent Q Sepharose 4 FastFlow. Macromolecular substances not included in the pores of the sorbent were eluted with the first peak (they include non-replicating nanoparticles), impurities were eluted after the peak of nonreplicating nanoparticles. Chromatographic conditions: flow rate 130 ml/min, buffer (10 mM TrisHCl, 75m M NaCl, 1 mM MgCl₂, 5% sucrose, 0.05% polysorbate 80, pH 8.0).
   Ethanol was added to obtained eluate (80 ml) to a concentration of 0.5%, and ethylenediaminetetraacetic acid (EDTA) to a concentration of 100 mcM, and then forwarded to the next stage.
8) Normal filtration.

The filtration was carried out through a filtering system with pore sizes of 22 µm to sterilize the resulting formulation. The final volume of the preparation at this stage was 80 ml and contained non-replicating nanoparticles with inset of influenza virus hemagglutinin gene of the same type at a titer of 3,3x10¹¹ PFU/ml. It was diluted with formulating buffer (e.g. 10 mM TrisHCl, July 5 mM NaCl, 1 mM MgCl₂, 5% sucrose, 0.05% polysorbate 80, 0.5% ethanol, 100 microns EDTA, pH 8.0) to obtain the stated activity 10⁷ PFU per 0.1 ml, which permitted long-term storage of the formulation at a temperature from +4 to +6°C without loss of its activity.

For the formulation of the final vaccine to 1-20 micrograms of immunostimulant (Russian Patent No 2195308), contained in 0.1 ml of distilled water, added the following: preparations of 3 types of non-replicating nanoparticles with inset of influenza virus hemagglutinin gene (from the list: H1, H3, HB) obtained in the previous stage with an activity of not more than 10⁷ PFU, and 0.1 ml of each preparation (i.e., total of 0.3 ml added) and formulating buffer adjusted to 0.5 ml volume. Then they were sterilized with normal filtration. Thus, the tast of vaccine preparation was solved.

### Example 3

Determination of an optimal dose of non-replicating nanoparticles.

Determination of dose of non-replicating nanoparticles was performed by evaluating the immunogenic and protective activity of non-replicating nanoparticles expressing genes of 3 types of influenza virus hemagglutinin.

Balb/c mice weighing 7.9 grams were used as experimental animals. For immunization of laboratory mice, the animals were divided into groups of at least 10 animals, immunization was carried out under light ether anesthesia. The group of mice receiving 0.9% solution of NaCl was used as a control group. Infection of animals with avian influenza or blood sampling for sera analysis was performed in three weeks after immunization.

The mice were immunized once intranasally with non-replicating nanoparticles at a dose of 10⁵ and 10⁷ PFU per animal in order to determine the abovementioned optimal dose of non-replicating nanoparticles based on the genome of human adenovirus type 5 expressing influenza virus haemagglutinin gene A/California/07/09 (H1N1). The volume of injected solutions was 50 microliters. Three weeks after immunization the blood samples were taken in animals in order to determine serum level of antibodies to the influenza virus A/California/07/09 (H1N1) and then the animals were exposured with the lethal dose of influenza virus H1 N1, adapted for mice.

Levels of specific antibodies to the influenza virus H1N1 were determined by routine virus neutralisation test (VNT) and hemagglutination inhibition (HAI).

Assessing the level of virus-neutralizing antibodies against H1N1 influenza virus in sera of immunized mice was determined by standard technique. According to the virus-neutralization reaction, increase of titers up to 1/10 was observed in the group of animals immunized with non-replicating nanoparticles at a dose of 10⁵ PFU per animal, which means the immunogenicity of a given dose. There was an increase in antibody levels within the limits of 1/10-1/20 titles was observed in animals treated with non-replicating nanoparticles at a dose of 10⁷ PFU per animal, which means the immunogenicity of a given dose.

The level of specific antibodies to H1N1 influenza virus hemagglutinin in the serum of animals immunized with the vaccine was determined using standard HAI techniques (hemagglutination inhibition).

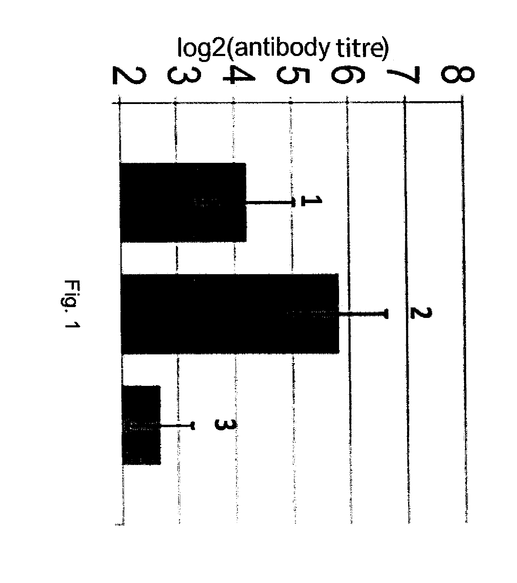

Figure 1 shows HAI results:
1 - the level of specific antibodies to influenza virus hemagglutinin H1N1 in the sera of mice intranasally immunized with non-replicating nanoparticles with the insert of hemagglutinin gene in the doses of 10⁵ PFU per animal;
2 - the level of specific antibodies to influenza virus hemagglutinin H1N1 in the sera of mice intranasally immunized with non-replicating nanoparticles with the insert of hemagglutinin gene in the doses of 10⁷ PFU per animal;
3 - control substance (0.9% solution of NaCl).

Thus, antibodies titer to hemagglutinin of avian influenza virus in sera of mice receiving non-replicating nanoparticles at a dose of 10⁵ PFU per animal was 4,2 log₂ (average geometric titer, AGT 1/20). Antibodies titer to hemagglutinin of avian influenza virus in sera of mice receiving non-replicating nanoparticles at a dose of 10⁷ PFU per animal was 5,8 log₂ (average geometric titer, AGT 1/64). In case of vaccination with control substance the titer was 2,7 log₂ ie AGT less than 1/10, which means no immunogenicity.

The titer of 10⁷ PFU per mouse was slightly more immunogenic compared to titer of 10⁵ PFU per mouse and VNT and HAI.

To study protective (safety) properties of mice vaccination with non-replicating nanoparticles at two different doses the immunized animals were exposured with a lethal dose of influenza virus H1N1. Infection was performed 21 days after intranasal immunization under light ether anesthesia. The animals were observed during 14 days after infection.

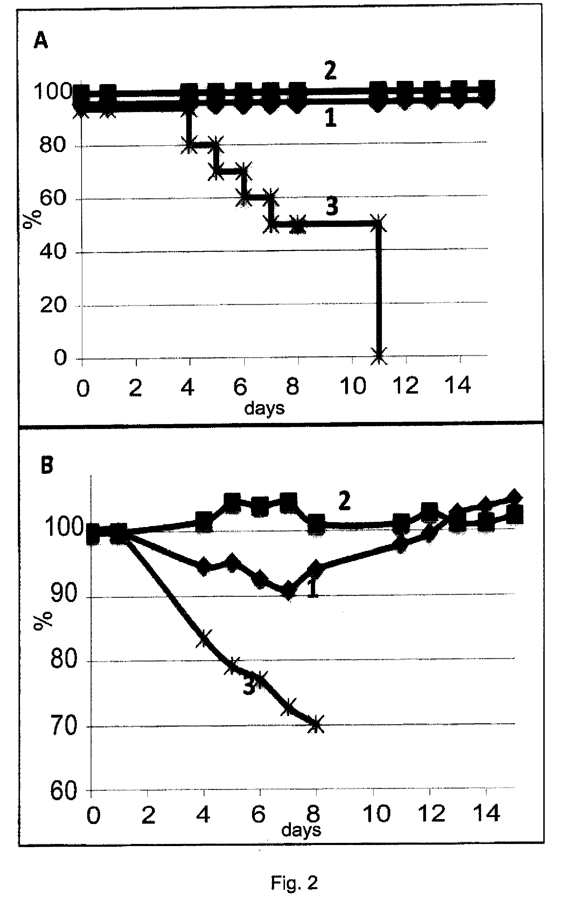

Figure 2 shows survival curves of mice (A) and changes in their body weight (B) after infection with a lethal dose of influenza virus H1N1:
Curve 1 ( 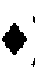 ) - Immunization of mice at a dose of 10⁵ PFU per animal;
Curve 2 ( 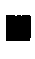 ) - immunization of mice at a dose of 10⁷ PFU per animal;
Curve 3 ( 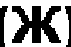 ) - a control substance (0.9% solution of NaCl).

Thus, the results presented in the figure show that mice intranasally immunized with non-replicating nanoparticles at a dose of 10⁵ PFU per animal, as well as at a dose of 10⁷ PFU per animal are 100% protected from death and symptoms of the disease (sudden weight decrease).

Non-replicating nanoparticles administered at a dose higher than 10⁷ PFU per animal caused undesirable local reactions and were relieved in the beginning of the experiment.

Thus, immunogenic dose of non-replicating nanoparticles in amount of not more than 10⁷ PFU per animal was selected for vaccine formulation.

### Example 4

Determination of an optimal dose of immunostimulant.

Acidic peptidoglycan with a molecular weight of 1,200 to 40,000 kDa (Russian Patent No 2195308) was chosen to enhance the immunogenicity of the selected in the previous example dose of nonreplicating nanoparticles carrying the influenza virus hemagglutinin gene, and for increase of anti-infective spectrum of action of the vaccine.

Determination of the optimal dose of immunostimulant was performed through one intranasal immunization of laboratory mice Balb/c weighing 7-9 g with nonreplicating nanoparticles at a dose of 10⁷ PFU per animal. Thus three doses of immunostimulant were used: 1, 10 and 20 µg per mouse. The total volume of the mixture of nonreplicating nanoparticles and immunostimulant introduced to animals was 50 microliters.

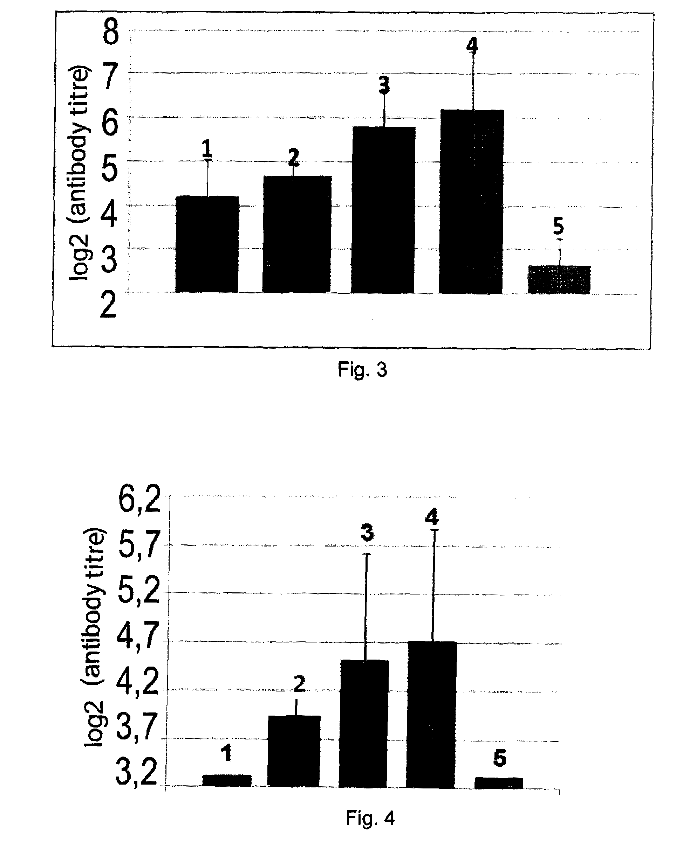

Three weeks after immunization the blood samples were taken in animals and the level of antibodies to influenza virus A/California/07/09 (H1N1) was evaluated using standard HAI techniques (Hemagglutination inhibition) and virus neutralisation test (VNT). Results are shown in Figures 3 and 4.

Figure 3 represents the levels of specific antibodies to the influenza virus hemagglutinin H1 N1 determined by HAI in sera of mice intranasally immunized with non-replicating nanoparticles at a dose of 10⁷ PFU per animal in conjunction with an immunostimulant at doses:
1 - without immunostimulant;
2 - 1 µg per animal;
3 - 10 µg per animal;
4 - 20 µg per animal;
5 - control substance (0.9% solution of NaCl).

Figure 3 shows a significant increase in the immunogenicity of nonreplicating nanoparticles in admixture with the immunostimulant at all proposed doses - 1, 10 and 20 micrograms per animal compared with nanoparticles administered without immunostimulant, as well as the control substance.

Figure 4 represents the levels of specific antibodies to the influenza virus hemagglutinin H1N1 determined by VNT in sera of mice intranasally immunized with non-replicating nanoparticles at a dose of 10⁷ per animal in conjunction with an immunostimulant at doses:
1 - without immunostimulant;
2 - 1 µg per animal;
3 - 10 µg per animal;
4 - 20 µg per animal;
5 - control substance (0.9% solution of NaCl).

Figure 4 also shows a significant increase in the immunogenicity of nonreplicating nanoparticles in admixture with the immunostimulant at all proposed doses - 1, 10 and 20 micrograms per animal compared with nanoparticles administered without immunostimulant, as well as the control substance (it reflects the background antibodies level).

Similar results (increased immunogenicity) of the above experiment were obtained by using bacterial cell wall peptidoglycan (drug "Glycopin") as an immunostimulant at a dose of 1, 10 and 20 micrograms per mouse.

Thus, mice treated with an immunostimulant at the doses of 1, 10 and 20 micrograms per mouse showed significant increase of antibodies as compared with the group receiving no immunostimulant, both in HAI and VNT analysis.

Thus, immunostimulant doses were determined in the range of 1-20 µg which effectively increase immunogenicity of non-replicating nanoparticles in a pharmaceutical composition.

### Example 5

### Determination of an optimal vaccine volume.

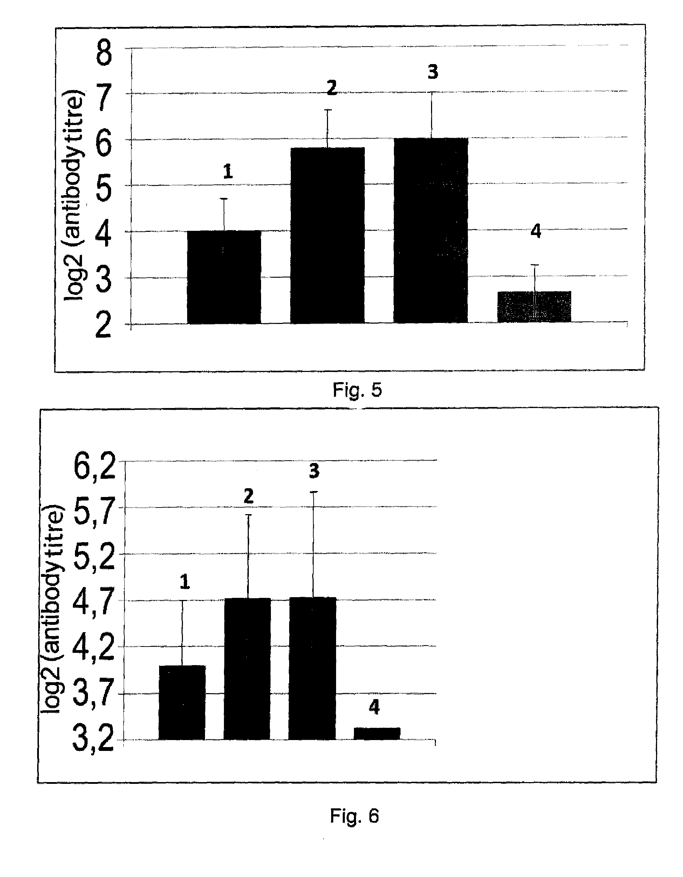

Determination of the optimal volume of intranasal immunization was performed through one intranasal administraiton of nonreplicating nanoparticles at a dose of 10⁷ PFU per animal and immunostimulant at a dose of 20 µg per animal to laboratory mice Balb/c weighing 7-9 g. Besides, this vaccine with immunostimulant in total amount of 5, 20 and 50 µl was halfed and administered into each nostril. Blood samples were taken in mice three weeks after immunization and determined levels of antibodies to the influenza virus using the methods of hemagglutination inhibition (HAI) and virus neutralization (VNT). Results of the experiment are shown on Figures 5 and 6.

Figure 5 represents the levels of specific antibodies to the influenza virus hemagglutinin H1N1 determined by HAI in sera of mice intranasally immunized with vaccine containing non-replicating nanoparticles at a dose of 10⁷ PFU per animal in conjunction with an immunostimulant at doses of 20 µg per animal, i.e. the researched volumes contained equal number of active substances. Vaccine was administered to mice in the amounts:
1 - 5 µl;
2 - 20 µl;
3 - 50 µl;
4 - control substance (0.9% solution of NaCl).

Figure 5 shows that the highest immunogenicity were observed in vaccines with the volume of 20 and 50 microliters.

Figure 6 represents the levels of specific antibodies to the influenza virus hemagglutinin H1N1 determined by VNT in sera of mice intranasally immunized with vaccine containing non-replicating nanoparticles at a dose of 10⁷ PFU per animal in conjunction with an immunostimulant at doses of 20 µg per animal. Vaccine was administered to mice in the amounts:
1 - 5 µl;
2 - 20 µl;
3 - 50 µl;
4 - control substance (0.9% solution of NaCl).

Figure 6 shows that the highest immunogenicity were observed in vaccines with the volume of 20 and 50 microliters.

Thus, 5 mcl volume of vaccine is insufficient for effective penetration of nonreplicating nanoparticles in airway cells and for the formation of immune response against influenza virus. Besides, immunization both in the volume 20 and in the volume of 50 µl was quite effective for the formation of high levels of antiinfluenza antibodies. Similar immunogenicity results showed the pharmaceutical composition of the same content, but with the amount of the immunostimulant of 1 µg.

For the convenience of use, 20 µl of the vaccine was considered as the optimal volume for the immunization of mice, and 50 µl (i.e. 0.5 ml) of the vaccine was considered as the optimal volume for the immunization of human (which coincides with the volume of majority known nasal influenza vaccines).

Thus, the optimal quantity of mixture components for immunization of laboratory mice against influenza is the amount of nonreplicating particles expressing the gene of influenza virus hemagglutinin at a dose of 10⁷ PFU per animal in conjunction with an immunostimulant at doses of 1 - 20 µg per animal in a volume of 50 µl (the mixture was adjusted with the formulating buffer during preparation).

### Example 6

Determination of immunogenic and protective properties of the trivalent vaccine with the immunostimulant.

After determination of the ratios of individual components of the vaccine in Examples 3, 4, 5, the researchers confirmed the immunogenicity of vaccine containing 10⁷ PFU of each of the 3 types of nonreplicating nanoparticles carrying different haemagglutinins of influenza virus (H1, H3, HB).

Immunogenicity of such trivalent vaccine with immunostimulant was studied in females of mice of Balb/c line weighing 9.7 grams intranasally immunized at first day with 20 mcl of vaccine containing 10⁷ PFU of activity of each type of non-replicating nanoparticles expressing influenza virus haemagglutinin gene and 20 µg of immunostimulant. 20 µl of physiological saline intranasally was administered to control group of mice. Taking of biological material (blood) was carried out in 5 mice in each group on day 21 after immunization. The titers of hemagglutinating antibodies were determined by HAI method (hemagglutination inhibition) to the strains of influenza virus A/California/07/09 (H1N1), A/Perth/16/2009 (H3N2), B/Brisbane/60/2008. Table 1 shows titers of specific antibodies in the serum on day 21 after immunization of mice with trivalent vaccine with an immunostimulant.

**Table 1**

| | **Haemagglutinating antibodies in HAI (average geometric titer)** |
|---|---|
| Against influenza virus A/California/07/09 (H1N1) | 970,1 ± 350,5 |
| Against influenza virus A/Perth/16/2009 (H3N2) | 5120 ± 0,0 |
| Against influenza virus B/Brisbane/60/2008 | 430,5 ± 362,0 |
| Against influenza virus in non-immunized mice (control group) | <8 |

According to the results of Table 1 we can see that the average geometric titer of hemagglutinating antibodies in HAI on Day 21 to A/California/07/09 (H1N1), A/Perth/16/2009 (H3N2), and B/Brisbane/60/2008 in immunized mice was significantly higher than in animals from control group.

The method described above was also used for testing the vaccine containing 10⁷ PFU of activity of each type of nonreplicating nanoparticles expressing influenza virus haemagglutinin gene and 20 µg of immunostimulant. Also a vaccine containing 10⁷ PFU of activity of each type of non-replicating nanoparticles with inset hemagglutinin gene and 1 mcg of immunostimulant was tested. In both cases a slightly smaller in comparison with examples described above but in general sufficient immunogenicity of compositions was shown.

Thus, according to HAI the dose of 3 types of non-replicating nanoparticles expressing multiple influenza virus haemagglutinin with not more than 10⁷ PFU of activity of each of them in the vaccine is sufficient for forming a good-specific humoral response in animals.

On day 21 after immunization the mice were infected with a lethal dose of influenza virus to assess the protection level of immunity formed after the injection of trivalent vaccine: Group 1- A/California/07/09 (H1N1), second group - Aichi/2/68 (H3N2), the third group - B/Brisbane/60/2008, adapted for mice. Three control groups were separately challenged with the same strain, but the mice were not vaccinated before challenge. After exposure of laboratory mice the follow-up period was conducted for 14 days. Survival rate and weight decrease of animals were recorded.

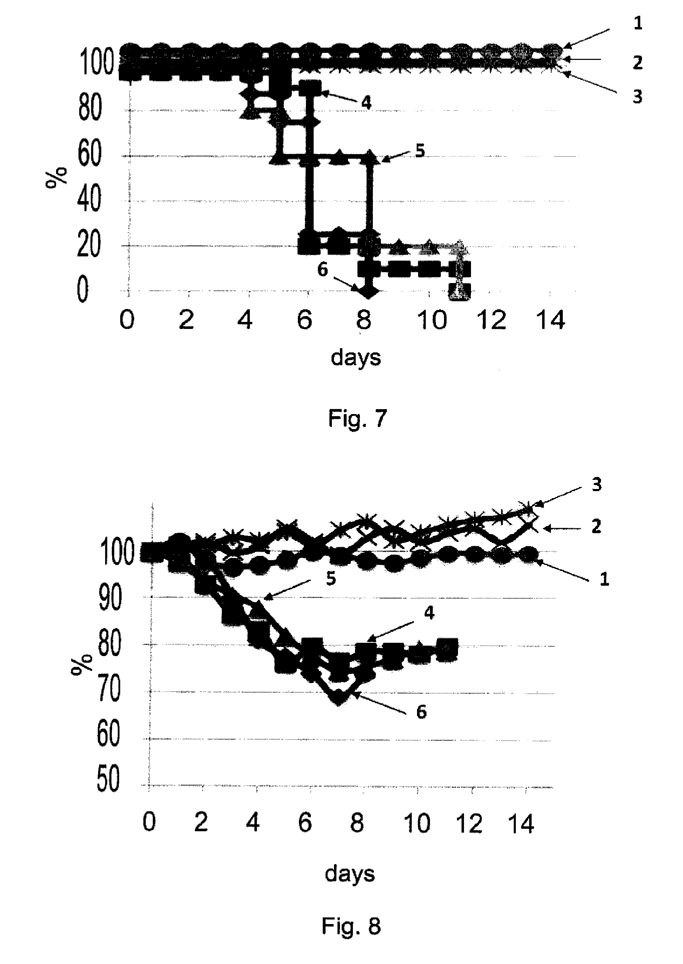

Figure 7 shows the survival curves of mice immunized with a trivalent vaccine after challenge with lethal doses of influenza A virus - H 1 N 1, H3N2, and influenza B. Curves and the names of the strains used for mice infestation:
1 (●)- A/California/07/09 (H1N1) (vaccine);
2 (X)-Aichi/2/68(H3N2) (vaccine);
3 ( 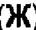 )-B/Brisbane/60/2008 (vaccine).
4 (■)-A/California/07/09 (H1N1) (control group);
5 (▲)-Aichi/2/68(H3N2) (control group);
6 (◆)-B/Brisbane/60/2008 (control group);

Figure 8 shows the weight loss curves of mice immunized with a trivalent vaccine after challenge with lethal doses of influenza A virus - H1N1, H3N2, and influenza B. Curves and the names of the strains used for mice infestation:
1 (●) A/California/07/09 (H1N1) (vaccine);
2 (X) - Aichi/2/68(H3N2) (vaccine);
3 ( 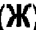 ) - B/Brisbane/60/2008 (vaccine).
4 (■) A/California/07/09 (H1N1) (control group);
5 (▲) - Aichi/2/68(H3N2) (control group);
6 (◆) - B/Brisbane/60/2008 (control group);

Figures 7 and 8 show that mice immunized with a trivalent vaccine were completely protected against infection with high doses of influenza viruses, and do not lose weight during the entire period of observation, as opposed to control animals.

A vaccine containing 10⁵ PFU of activity of each type of non-replicating nanoparticles with insert of haemagglutinin gene and 20 µg immunostimulant and a vaccine containing 10⁷ PFU of activity of each type of nonreplicating nanoparticles with insert of haemagglutinin gene and 1 µg immunostimulator were also investigated by the method described above for immunogenicity. The results obtained were similar to those shown in Figures 7 and 8.

Thus animals receiving saline died completely within 11 days after infection with any of the 3 viruses.

Thus, it was shown that a single intranasal immunization of laboratory mice with the trivalent vaccine with immunostimulant leads to the induction of a protective immune response against infection with high doses of influenza viruses. Besides, the immunized mice are completely protected from infection with lethal doses of relevant influenza virus strains.

### Example 7

Determination of broad antimicrobial properties of immunostimulant in the content of the vaccine.

To determine the ability to activate innate immunity and, accordingly, broad antimicrobial protection of immunostimulant in the content of the vaccine, the researchers evaluated the immunostimulant ability to specifically bind to human Toll-like (TLR) receptors in the conditions in vitro (Tukhvatulin Al et al, Toll-like receptors and their adapter molecules, Biochemistry, 2010, vol.75, No 9, p. 1224 - in Russian). The experiment used standard techniques with the use of set of eukaryotic cell lines based on human embryonic kidney epithelial cell line (HEK293) expressing different types of Toll-like receptors.

Commonly the cell of HEK293 lines do not express human Toll-like receptors. The aforesaid property of HEK293 line cells allows to create on their basis a set of sublines, in which genes of different human Toll-like receptors were inserted using genetic engineering methods. Furthermore, β-galactosidase reporter gene under control of NF-kB-dependent promoter was inserted in the genome of the cells to detect ligand interaction with Toll-like receptor and for activation of the latter.

The initiation of underlying intracellular signaling cascade is performed upon binding of the ligand added to the cells with corresponding Toll-like receptor on the surface of described cells, leading ultimately to activation of the transcription factor NF-kB, which then migrates into the nucleus and binds to own promoter's area, starting expression of β-galactosidase reporter gene.

This scheme allows quantifying of ligand interaction with the corresponding Toll-like receptor and its subsequent activation using colorimetric method. In the course of this method a colorless substrate β-galactosidase-O-nitorphenylgalactopiranoside (ONPG) is added in cell lysis buffer, which under enzyme cleavage is converted into a colored product. The color intensity (optical density) of the solution is directly proportional to the intensity of activation of Toll-like receptor, activation of the transcription factor NF-kB and further expression of β-galactosidase reporter gene.

3 sublines of cells were used (HEK293- hTLR2/CD14, HEK293-hTLR4/CD14-MD2, HEK293-hTLR5) expressing following types of Toll-like receptors: TLR2/CD14, TLR4/CD14-MD2, TLR5, respectively.

To confirm the specificity of research sample molecules interaction with Toll-like receptors, the experiment used control cells that do not express Toll-like receptor but contain in its genome β-galactosidase reporter gene under the control of NF-kB-dependent promoter - HEK293-hTLRnull.

Absence of cytopathic effect of test samples, which may cause the lack of NF-kB activation and as a consequence the reduced level of β-galactosidase expression, was determined using the method of determination of survival rate of eukaryotic cells by staining them with methylene blue colorant.

Cells of lines HEK293-hTLR2/CD14, HEK293-hTLR4/CD14-MD2, HEK293-hTLR5, HEK293-hTLRnull were passaged on 25 cm² culture mattress in a culture media DMEM with 10% of fetal serum at 37°C, 5% CO2. For the experiment the cells were dispersed in 96 well plate at the rate of 2*10⁴ cells per well. Media volume in the well was 100 microliters. After sieving of cells the plate was incubated at + 37°C and 5% CO₂ for 18-24 hours.

The next day, the test samples of immunostimulant in a volume of 10 µl (1/10 of the original volume of well medium) with different concentrations were added to the cells. The following working immunostimulant concentrations were used in both experiments: 50 µg/ml, 5 µg/ml, 500 ng/ml, 50 ng/ml, 5 ng/ml, 500 pg/ml, 50 pg/ml. Intact cells were used as negative control (C-) in the experiment. The cells with addition of known ligands of corresponding-like receptors (lipopolysaccharide (LPS) E. coli - ligand of Toll-like receptor 4, a synthetic lipopeptide (Rpam) - ligand of Toll-like receptor 2, flagellin protein - ligand of Toll-like receptor 5) were used as a positive control, confirming the functionality of the Toll-like receptors expressed by different cell lines. In the case if control cells did not express Toll-like receptor cells (HEK293-hTLRnull), the cells with addition of tumor necrosis factor alpha (TNF) which is able to activate the transcription factor NF-kB binding to its own receptor (TNFR) on the surface of the cell line HEK293, were considered as positive control. The expression of β - galactosidase level and determination of dose toxicity for analysed sample using methylene blue colorant was measured on the third day after the addition of the drug.

Activity of the immunostimulant included in the vaccine to the following types of Toll-like receptors: TLR2/CD14, TLR4/CD14-MD2 and TLR5 were determined by the results of the experiment.

Thus, an immune stimulant, which is part of the vaccine activates Toll-like receptors (TLR), which are one of the key receptors of mammal innate immune system, activation of which determines the presence of a wide spectrum of antimicrobial action. The task of giving the vaccine the ability to activate innate immunity was achieved.

### Example 8

Clinical approbation of recombinant trivalent vaccine was carried out on 2 groups of volunteers, each containing 10 healthy non-immune to influenza virus people (the third group - control group received 0,9% solution of NaCl). The vaccine contained non-replicating nanoparticles expressing influenza virus haemagglutinin of each of three types:

The vaccine of a volume of 50 µl contained 10⁷ PFU of each of the 3 types of non-replicating nanoparticles with haemagglutinin gene insert (H1, H3, HB) and 20 µg of immunostimulant. Half of this amount of the vaccine (i.e., 25 mcl) was administered by infusion in each nostril using a sterile pipette. Single administration was performed. Blood samples were taken in all immunized patients on the day of vaccination and after 21 days for evaluation of the presence of antibodies in a standard HAI (hemagglutination inhibition) to a specific antigen which was part of the vaccine. It is known that a protective level of hemagglutinating antibodies capable of creating a protection against influenza disease in 50% of people comprises ≥ 40 (Coudeville L. et al. Relationship between haemagglutination inhibiting antibody titres and clinical protection against influenza: development and application of a bayesian random-effects model, BMC Medical Research Methodology, 2010; http://www.biomedcentral.com/1471-2288/10/18.). The results are shown in Table 2.

**Table 2**

| **Types of expressed hemagglutinin by influenza vaccine** | **Titers % ≥ 40** | | |
|---|---|---|---|
| | **H1** | **H3** | **HB** |
| H1, H3, HB | 80,9 ± 0,2 | 83,6 ± 0,3 | 88,3 ± 0,5 |
| 0.9% NaCl solution (Reference substance) | 0 | 0 | 0 |

Thus, the results in the table show the formation of a protective level of specific hemagglutinating antibodies to each of the types of recombinant hemagglutinin expressed by non-replicating nanoparticles of trivalent vaccine in the majority of people (more than 80,9 ± 0,2%).

Protective levels of specific antibodies to the influenza virus hemagglutinin kept stable for 6 months or more. Blood samples for HAI were taken at 0, 2, 5, 16, 24 and 28th week; the titers were respectively <1/10, 1/20, 1/500, 1/500, 1/200, 1/100.

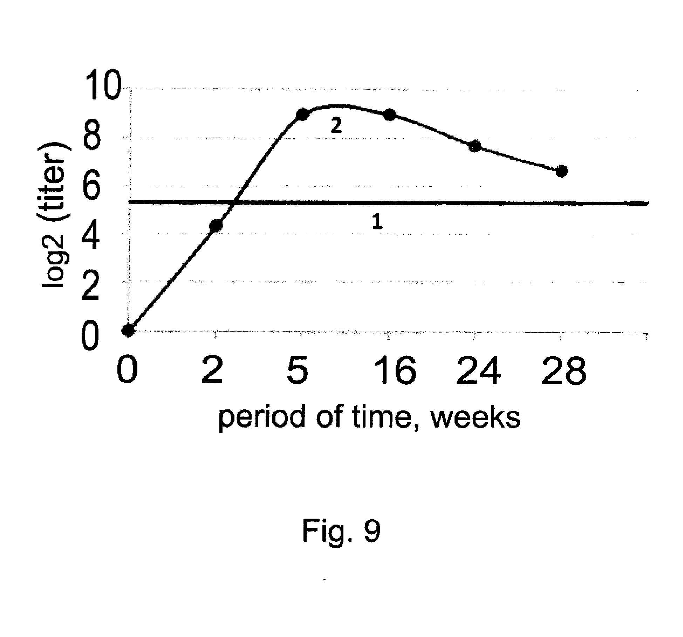

Figure 9 shows level curves of specific antibodies to the influenza virus hemagglutinin H1. Curves:
1 (-) - protective level of hemagglutinating antibodies;
2 (●) -level of antibodies to H1.

On Figure 9 the curve of hemagglutinating antibody level rises above the known protective level of hemagglutinating antibodies starting from third week and stays higher at least until the 28th week, what indicates the availability of protection against the influenza virus for at least 6 months.

Specific hemagglutinating antibody titers to recombinant H3 and HB showed similar dynamics to the one stated on Figure 9, i.e. they were also protective for at least 6 months.

Thus, as a result of immunization with recombinant trivalent vaccine with immunostimulant the antibody titers in the HAI obtained to each of three expressed hemagglutinin nanoparticles in its content were protective in the majority of people, allowing to use a vaccine to generate wide immune coverage in epidemiological indications. Administration of recombinant trivalent vaccine with immunostimulant to human did not cause any local or systemic vaccine-related side effects and was easily tolerated by the body.

Thus, compared with the prototype, the advantages of the claimed recombinant trivalent vaccine containing non-replicating nanoparticles based on human adenovirus genome of 5 serotype and producing different types of hemagglutinin of influenza virus and an immunostimulant are:
- A single administration for a protective immune response in humans within 6 months or more to all forming parts of the vaccine strains of influenza virus;
- Lower costs of drugs, medical instruments, working time of medical staff;
- The ability to quickly create a 3-valent vaccine from seasonal epidemiologically significant strains;
- Reduction of titer of nonreplicating nanoparticles with inset of hemagglutinin gene required to establish protective immunity in humans, 10 times for each strain contained in the vaccine;
- Reduction of vaccine reactogenicity;
- Reduction of production cost;
- A wide range of preventive antimicrobial action directed against infectious diseases of the respiratory tract.

### INDUSTRIAL APPLICABILITY

The above described examples, tables and figures confirm the industrial applicability and task performance, namely: the creation of 3-valent vaccine which in single intranasal administration will provide protective immune response in human for more than 6 months to all strains of influenza virus contained in the vaccine; reduction of the cost of production and the cost of drugs, medical instruments and medical staff working hours allowing a rapid creation of vaccine from seasonal epidemiologically significant strains; reduction of vaccine reactogenicity; providing a wide spectrum of antimicrobial prophylactic vaccines directed against infectious diseases of the respiratory tract.

## Claims

1. Recombinant trivalent vaccine against human influenza comprising a recombinant adenoviral vector with a nucleotide sequence which encodes at least one antigen of the influenza virus strain, wherein non-replicating nanoparticles are used as a adenoviral vectors based on genome of human adenovirus serotype 5, capable of expressing the haemagglutinin of influenza virus inducing immune response in human to influenza virus, further comprises a composition of three types of non-replicating nanoparticles, each of which carries different influenza virus hemagglutinin genes and also contains an additional immunostimulant and formulating buffer.

2. Recombinant trivalent vaccine according to claim 1, wherein the genes of the influenza virus hemagglutinin are H1, H3, HB.

3. Recombinant trivalent vaccine according to claim 2, wherein the genes of the influenza virus hemagglutinin H1, H3, HB are any genes recommended by the World Health Organization at the time of production.

4. Recombinant trivalent vaccine according to claim 1, wherein formulating buffer is added in the composition to achieve a total volume of 0.5 ml.

5. Recombinant trivalent vaccine according to claim 4, wherein the vaccine dose is 0.5 ml.

6. Recombinant trivalent vaccine according to claim 5, wherein it comprises:
- Non-replicating nanoparticles with insert of hemagglutinin H1 not more than 10⁷ PFU;
- Non-replicating nanoparticles with insert of hemagglutinin H3 not more than 10⁷ PFU;
- Non-replicating nanoparticles with insert of hemagglutinin HB not more than 10⁷ PFU;
- Immunostimulant 1 - 20 µg

7. Recombinant trivalent vaccine according to claim 1, wherein peptidoglycans are used as an immunostimulant.

8. Recombinant trivalent vaccine according to claim 7, wherein the acidic peptidoglycan is used as peptidoglycan having a molecular weight from 1,200 to 40,000 kDa.

9. Recombinant trivalent vaccine according to claim 7, wherein the peptidoglycan of the bacterial cell wall is used as peptidoglycan.

10. Recombinant trivalent vaccine according to claim 5, wherein the vaccine administered intranasally.
